# EUROPEAN PATENT APPLICATION

(11) **EP 4 671 228 A1**
(43) Date of publication of application: **31.12.2025**
(21) Application number: 25185328.9
(22) Date of filing: 25.06.2025
(51) Int. Cl.: C05F 5/00, A01N 63/32, C05F 11/08, C05G 3/60, C05G 5/23, C12N 1/16, C05F 11/10

(54) **PROCESS FOR INDUCING THICKENING OF THE SKIN OF GRAPE BERRIES IN A VINE CULTIVATION AND USE OF AN INACTIVATED YEAST OR DERIVATIVE THEREOF FOR TREATING A VINE CULTIVATION, IN PARTICULAR FOR INDUCING THICKENING OF THE SKIN OF GRAPE BERRIES**

(30) Priority: 25.06.2024 IT 202400014518
(71) Applicant: ENOLOGICA VASON S.P.A., 37029 San Pietro in Cariano (VR) (IT)
(72) Inventor: VASON, Albano, 37029 San Pietro in Cariano (VR) (IT); ZANELLA, Gianmaria, 37015 Sant'Ambrogio di Valpolicella (VR) (IT); GIOTTO, Federico, 31051 Follina (TV) (IT)
(74) Representative: Gallo, Luca

(57) **Abstract**

Process for inducing thickening of the skin of grape berries in a vine cultivation, which involves inserting an active yeast for winemaking use with a water residue of at least 40% into a pressurization chamber, inside which a fluid is forced at a lysis pressure greater than 1000 bar to subject the active yeast to said lysis pressure with consequent breakage of the molecular structure of the yeast and obtaining an inactivated yeast or derivative thereof containing a free amino acids fraction extracted from the cytosol of the same yeast. The depressurization of the pressurization chamber then takes place and the inactivated yeast or derivative thereof is extracted from the pressurization chamber itself.

Furthermore, the method involves dissolving the inactivated yeast or derivative thereof in a solvent in order to obtain a treatment solution, distributing the treatment solution on at least one vine plant of the vine cultivation and allowing the vine plant to absorb the free amino acids fraction extracted from the cytosol.

## Description

### Field of application

The present invention relates to a process for inducing thickening of the skin of grape berries in a vine cultivation and the use of an inactivated yeast or derivative thereof for treating a vine cultivation, in particular for inducing thickening of the skin of grape berries, according to the preamble of the respective independent claims. The process in question is intended to be used to advantageously treat the vine plants of a cultivation, i.e., of a vineyard.

In more detail, the process and use of an inactivated yeast or derivative thereof in question are advantageously usable for inducing thickening of the skin of grape berries. Furthermore, the process and use of an inactivated yeast or derivative thereof in question are advantageously intended for the improvement of vine plants, i.e., to improve the quality of the grape, in particular the phenolic and aromatic ripening thereof, as well as of the wine obtained therefrom, in particular the pleasantness of the taste and the organoleptic and compositional characteristics of musts and wines. Secondly, the process and use of an inactivated yeast or derivative thereof in question are advantageously adapted to obtain greater protection of the grapes during the ripening phase and to induce greater resistance in the vine plants against fungal attacks, in particular downy mildew.

The process and use of an inactivated yeast or derivative thereof in question therefore find optimal use in the viticulture sector and consequently in the winemaking sector of industrial wine production.

### State of the art

Currently, as is known, the winemaking process of grapes involves, after an initial phase of crushing and pressing the grapes, an alcoholic fermentation phase. In this phase, the sugars transform into alcohol. The alcoholic fermentation of musts can be obtained either through the natural microflora originally present in the grapes or through the introduction of specifically selected yeasts.

At the end of fermentation, the winemaking liquid is also subjected to further phases which can differ based on the variety of starting grapes and the type of wine to be obtained.

As is known, however, the quality and characteristics of the wine obtained at the end of the winemaking process do not depend only on the operations and situations in the cellar, but rather derive largely from the characteristics and quality of the grapes and the conditions thereof at the time of the harvest.

Therefore, the process of managing vineyards and, specifically, the care of the vine itself before the harvest are subjected to great attention, as they are particularly complex for obtaining the optimal quality of the fruit.

In more detail, if on the one hand the vine (common name of *Vitis vinifera,* a deciduous and liani-like plant belonging to the Vitaceae family) is a very resistant basic plant, particularly to drought, on the other hand it requires ideal nutritional conditions to produce high quality fruit.

For this reason, a particular fertilization regime is envisaged in the viticulture sector, which involves incorporating fertilizer with manure-based mixtures into the soil and/or administering chemical fertilizers in granular form under the rows.

Specifically, the use of particular classes of products, called soil improvers, is known, which consist of various substances, both natural and synthetic, mineral and organic, capable of modifying and enhancing the chemical, physical, biological and mechanical characteristics of a soil. Their main use is to improve soil structure and maintain the biological fertility of the soil.

For example, in addition to manure itself, the use of additives based on fermented sugar is known from patent US 2016068450, or, as described in patent RU 2661842, the use of compost obtained from vine pruning fermented with an oenological yeast mixed with grape must.

Furthermore, the recent use of products called Cerevisane as soil improvers is known. These products consist of an inert extract obtained from the cell walls of the yeast *Saccharomyces cerevisiae* belonging to the particular strain LAS117.

More in detail, the use of yeast cell walls is described in patent US 2009010905. In particular, according to the process illustrated in this document, the vine plants are sprinkled exclusively with yeast cell walls comprising at most insoluble fractions, so that the latter remain outside the plant and act as protection for it.

Other products are also known, called plant fortifiers, which are substances of natural origin capable of improving the resistance of plants against harmful organisms and protecting plants from non-parasitic damage. Furthermore, it is known that treatments are carried out using phytosanitary products or agrochemicals, i.e., synthetic or natural products used to treat plant diseases or which promote their vital processes (with the exception of manures and fertilizers).

Agrochemicals are divided into various types, such as fungicides or pesticides (against fungi), herbicides or weed killers (against weeds), insecticides and acaricides (against insects), nematicides and fumigants (for soil disinfestation), rodenticides (against harmful rodents) and molluscicides (against slugs and other molluscs).

For example, one of the best-known diseases of vines is grape downy mildew, a fungal disease caused by the fungus *Plasmopara viticola.* In particular, downy mildew is a fungus that develops in humid environments at mild or warm temperatures; it attacks the bunches, preventing their uniform development, and preventing correct ripening. Generally, downy mildew is fought by using synthetic chemical agents or by using copper.

All the treatments described above, which fall within the scope of the viticulture sector, have proven in practice to be not free from drawbacks.

A first drawback lies in the fact that lately there has been a particular need to implement organic viticulture and therefore not all of the above-mentioned products, particularly those of chemical synthesis, are permitted in such cultivations.

For example, in the treatment of downy mildew, most chemical agents are not permitted in organic farming, while copper is permitted only with a certain, more stringent number of doses and treatments permitted in a year and with particular, predefined methods.

A further drawback is that many of the chemical fertilizers and phytosanitary products have a significant environmental impact, in both the production and use thereof, and therefore the use thereof is increasingly limited and sometimes prohibited.

A further drawback, particularly related to phytosanitary products, lies in the fact that each thereof, in order to obtain marketing authorization from the European Commission and the Ministry of Health, must pass a long series of tests defined to evaluate and carefully examine the risks deriving from the use thereof. Specifically, the competent authorities evaluate the product's efficacy, chemical composition, environmental impact and health effects.

Therefore, the introduction of agrochemicals into the market is particularly complex and the development thereof requires an average time of about ten years overall, as well as significant investments.

A further drawback is that even the products currently known in the sector that can be used in organic farming, and in particular the Cerevisane mentioned above, have limited efficiency, as they are substantially derived from waste by-products from processing yeasts for winemaking use and are not optimized for this purpose. Furthermore, in addition to not having a proven efficacy, these latter products do not even have any nutritional purpose for the plant, as they necessarily remain on the outside and are quickly washed away by rain and other atmospheric agents.

Furthermore, there are currently no known procedures and treatments in the sector, particularly natural ones, capable of increasing the quality of grape berries, in particular the thickness of their skin, to give them better organoleptic properties and/or greater resistance, as well as to make them crunchier and more appealing to the consumer.

### Presentation of the invention

The essential object of the present invention is therefore to overcome the drawbacks manifested by the known solutions mentioned above by providing a process for inducing thickening of the skin of grape berries in a vine cultivation and the use of an inactivated yeast or derivative thereof for treating a vine cultivation, in particular for inducing thickening of the skin of grape berries, which are capable of inducing or promoting thickening of the skin of grape berries without using phytosanitary products.

A further object of the present invention is to provide a process for thickening of the skin of grape berries in a vine cultivation and the use of an inactivated yeast or derivative thereof for treating a vine cultivation, which are capable of improving the quality and organoleptic properties of the grapes, inducing a better phenolic and aromatic maturation.

A further object of the present invention is to provide a process for thickening of the skin of grape berries in a vine cultivation and the use of an inactivated yeast or derivative thereof for treating a vine cultivation, which allow for a greater pleasantness in the taste of the wines obtained from the treated grapes.

A further object of the present invention is to provide a process for thickening of the skin of grape berries in a vine cultivation and a use of an inactivated yeast or derivative thereof for treating a vine cultivation, which have a low environmental impact.

A further object of the present invention is to provide a process for thickening of the skin of grape berries in a vine cultivation and the use of an inactivated yeast or derivative thereof for treating a vine cultivation, which are particularly suitable also for organic viticulture with greater efficacy than the currently known products.

A further object of the present invention is to provide a process for thickening of the skin of grape berries in a vine cultivation and the use of an inactivated yeast or derivative thereof for treating a vine cultivation, which are capable of increasing the protection of the grapes during the ripening phase.

A further object of the present invention is to provide a process for thickening of the skin of grape berries in a vine cultivation and the use of an inactivated yeast or derivative thereof for treating a vine cultivation, which are capable of increasing the resistance of vine plants to fungal attacks, in particular downy mildew, powdery mildew and botrytis.

A further object of the present invention is to provide a process for thickening of the skin of grape berries in a vine culture and a use of an inactivated yeast or derivative thereof for treating a vine culture that are easy to implement.

A further object of the present invention is to provide a process for thickening of the skin of grape berries in a vine cultivation and the use of an inactivated yeast or derivative thereof for treating a vine cultivation, which comply with the legislation in the viticultural and winemaking field and are completely reliable.

### Brief description of the drawings

The technical characteristics of the present invention, according to the aforesaid objects, can be seen in the contents of the claims reported below and the advantages thereof will be more evident in the detailed description that follows, made with reference to the experimental figures of the process according to the invention, in which:
- Figures 1A, 1B, 1C, 1D and 1E show figures related to comparative analyses of the effect on the sugar content of Merlot grapes from different vintages and cultivations treated with an example of the process of the present invention and with a control process without treatments;
- Figures 2A and 2B show figures related to comparative analyses of the effect on the evolution of the sugar content of Merlot grapes from different vintages treated with examples of the process of the present invention, with a process using a known product and with a control process without treatments;
- Figures 3A, 3B, 3C and 3D show figures related to comparative analyses of the effect on the sugar content of Corvina Veronese grapes from different vintages and cultivations treated with an example of the process of the present invention and with a control process without treatments;
- Figure 4 shows a figure related to a comparative analysis of the effect on the total acidity of Cabernet Franc grapes from the 2023 vintage treated with an example of the process of the present invention and with a control process without treatments;
- Figures 5A, 5B and 5C show figures related to comparative analyses of the effect on the total acidity and in particular on tartaric acid of Corvina Veronese grapes from different cultivations treated with an example of the process of the present invention and with a control process without treatments;
- Figures 6A and 6B show figures related to comparative analyses of the effect on the evolution of the acidity of Merlot and Chardonnay grapes treated with examples of the process of the present invention, with a process using a known product and with a control process without treatments;
- Figures 7A and 7B show figures related to comparative analyses of the effect on the ammonium nitrogen content of Corvina Veronese grapes from different cultivations treated with an example of the process of the present invention and with a control process without treatments;
- Figures 8A, 8B and 8C show figures related to comparative analyses of the effect on the anthocyanin and polyphenol content of Corvina Veronese grapes from different cultivations treated with an example of the process of the present invention and with a control process without treatments;
- Figures 9A, 9B and 9C show figures related to comparative analyses of the effect on the evolution of the anthocyanin and flavonoid content of Merlot grapes from different vintages treated with examples of the process of the present invention, with a process using a known product and with a control process without treatments;
- Figures 10A and 10B show figures related to comparative analyses of the effect on the anthocyanin and polyphenol content of Merlot grapes from different cultivations treated with an example of the process of the present invention and with a control process without treatments;
- Figures 11A, 11B, 11C, 11D, 11E and 11F show figures related to comparative analyses of the effect on the color of wines obtained from different grapes treated with an example of the process of the present invention, with a control process without treatments and in some cases with a process using a known product;
- Figures 12A, 12B, 12C and 12D show figures related to comparative analyses of the effect on the content of aromatic precursors in grapes treated with examples of the process of the present invention, with a process using a known product and with a control process without treatments;
- Figures 13A, 13B and 13C show figures related to comparative analyses of the effect on the sensory characteristics of wines obtained with grapes treated with examples of the process of the present invention, with a process using a known product and with a control process without treatments;
- Figures 14A, 14B, 14C, 14D, 14E and 14F show figures related to comparative analyses of the effect on the weight of the bunches of various grapes from different vintages and cultivations treated with an example of the process of the present invention and with a control process without treatments;
- Figures 15A and 15B show figures related to comparative analyses of the effect on the weight of the berries of Sangiovese grapes from different vintages treated with examples of the process of the present invention, with a process using a known product and with a control process without treatments;
- Figures 16A, 16B and 16C show figures related to comparative analyses of the effect on the weight of the berries of various grapes from different vintages and cultivations treated with an example of the process of the present invention and with a control process without treatments.

### Detailed description of a preferred form of the present invention

Generally speaking, a vine cultivation comprises a plurality of vine plants, which have an annual cycle comprising various phenological phases. In technical jargon, these phenological phases are called: planting, budding, vegetation, flowering, fruit setting, veraison, ripening and vegetative rest.

In more detail, the planting phase generally occurs around the month of March, when the ambient temperature increases from cold winter temperatures to milder spring temperatures. In this planting phase, part of the plant's sap leaks out following the pruning in winter. This leakage is a sign of the resumption of root activity, which allows the trunk and shoots to regenerate with the water and mineral salts lost during the winter.

Subsequently, in the budding phase, which occurs around the month of April, the buds swell, followed by the consequent appearance of the first leaves, which highlights the vegetative recovery.

Subsequently, the growth of leaves and the development of new shoots occurs in the vegetation phase. In particular, the vegetation phase begins in April and generally lasts until August, the period in which the shoot matures and its color changes from green to brown.

At the same time, generally between May and June, the flowering phase is expected, in which the flowers open. This flowering phase is highly dependent on climatic conditions and can be compromised in the event of sudden frosts or heavy rains.

Subsequently, generally a few days after flowering, between June and July, the fruit setting phase occurs, in which a grape arises from each flower. Furthermore, during the fruit setting phase, the grapes swell and the flowers that are not fertilized fall (a phenomenon called "shatter").

Generally, the veraison phase occurs between July and August, when the vegetative phases of the grapes mentioned above end and the ripening phase begins. During the veraison phase, the grapes change color and in the case of red berried grapes they become red or black (depending on the variety). On the contrary, in the case of white berried grapes they become yellow to green in color and have a much clearer transparency. Furthermore, during this veraison phase, the sugar and tartaric acid content inside the grapes begins to increase, while the malic acid content decreases.

In general, the ripening phase occurs between September and October and continues for a variable ripening period, which is between approximately 30 days (for early varieties) and 50-60 days (for late varieties). In particular, the ripening phase is considered complete when the grapes reach technological maturity, i.e., a predefined balance between sugars and acids, and phenolic maturity, i.e., the extractability of tannins and coloring substances contained mostly in the skin.

As is known, in the broader process of managing the vine cultivation, at the end of the ripening phase a harvest phase is carried out, in which the grapes are collected.

Finally, at the end of the harvest phase, a winter vegetative rest phase is envisaged. In this phase of vegetative rest, the vine reduces lymphatic activity and prepares for the next annual cycle. During the vegetative rest phase, the vine is pruned to maintain constant productivity and the proper balance between the vegetative activity of the plant and fruiting.

The process for inducing thickening of the skin of grape berries which is the subject matter of the invention is advantageously intended to be used to treat the vine plants of a corresponding cultivation during one or more of the phenological phases indicated above. In particular, as will be better illustrated below, the process in question is intended to be carried out at least in part after the phenological phase of fruit setting. Furthermore, the process is intended to be carried out preferably at least in part before the ripening phase stage and more preferably at least in part during the veraison phase.

The process for treating a vine cultivation advantageously involves the use of an inactivated yeast (or derivative thereof), i.e., more precisely, a yeast derivative, obtained in accordance with any form and embodiment of the method described in European patent EP 3561047, which shall be deemed to be attached by reference in the present description.

The term "inactivated yeast or derivative thereof" shall mean any product of the transformation and processing of inactivated yeast, for example an autolysed yeast (for example obtained with a variant of the method described below in which a higher lysis pressure or a longer lysis time is used), a yeast cell wall (for example obtained by a variant of the method described below, in which a specific filtration and purification step is added), a yeast hull (containing cell walls and membranes, for example obtained by a variant of the method described below, in which a non-specific filtration step is added), or a yeast extract (for example obtained by a variant of the method described below, in which at least the cell walls are separated). All these products of the transformation and processing of inactivated yeast are to be considered advantageously enriched with free amino acids extracted from the cytosol, and advantageously also from the nucleus, of the yeast, as will be better described below.

Preferably, the process and use which are the subject matter of the present invention involve the use of an inactivated yeast as is or of an autolysed yeast.

In fact, it has surprisingly been found, as will be better shown in detail below, in agreement with the reported experimental results, that an inactivated yeast or derivative thereof thus obtained has an important effect in inducing the thickening of the skin of grape berries.

Furthermore, it has surprisingly been found that such inactivated yeast or derivative thereof has important improvement effects on vine plants and allows improving the quality of the grapes. Furthermore, it has surprisingly been found that this inactivated yeast or derivative thereof allows inducing a greater resistance of the vine plant to fungal attack, in particular to downy mildew *(Plasmopora viticola*) and advantageously also to powdery mildew *(Erysiphe necator and Oidium tuckeri)* and botrytis *(Botrytis cinerea).*

Advantageously, these effects are mainly due to a greater availability of a free amino acids fraction, which is not present in the cell walls of traditionally used yeasts and which is absorbed by the plant when the inactivated yeast or derivative thereof is distributed on it.

Advantageously, this yeast is an inactivated yeast or derivative thereof in compressed or cream form, which can be used as is or dried (for example using techniques well known in the wine sector, such as cold freeze-drying or spray drying) and is unsuitable for carrying out fermentation activity, whereby such an inactive condition is achieved by means of the process phases described below and advantageously also described in the aforesaid European patent EP 3561047.

In more detail, in the context of the present invention, the term cream yeast (CRY) means a yeast with a dry matter content preferably comprised between 18 and 25% and a level of viable yeasts equal to or greater than 10¹⁰ CFU/g of dry matter. Furthermore, in this document, the term compressed yeast (COY) means a yeast with a dry matter content preferably comprised between 30 and 35% and a level of viable yeasts equal to or greater than 10¹⁰ CFU/g of dry matter. Advantageously, these definitions of cream and compressed yeast are compliant with the provisions of Resolution OIV (International Vine and Wine Organization) - OENO 576A -2017. According to the invention, the process in question provides for a step of arranging an active yeast for winemaking use, not dried, with water residue of at least 40% by mass. Preferably, the active yeast is a cream yeast with a water content of around 75% by mass, or it is a compressed yeast with a water content of around 55% by mass, and preferably it is a compressed yeast of the type described in European patent EP 1236795, to be understood as attached hereto by reference from paragraph [0036] to paragraph [0076].

In accordance with a further embodiment, the active yeast to be made available is a frozen yeast (AFY). In more detail, in the present document, the term "frozen yeast" means a yeast with a dry matter content preferably comprised between 40 and 85% and a level of viable yeasts equal to or greater than 10¹⁰ CFU/g of dry matter, as indicated in Resolution OIV - OENO 576A -2017.

According to the invention, the process provides for an inactivation step, in which the active yeast, preferably in the forms described above, and a pressurized fluid are inserted into a pressurization chamber. In particular, the pressurized fluid has a lysis pressure greater than 1000 bar, to subject the active yeast to the lysis pressure with consequent breakage of the cell structure of the active yeast and release of a free amino acids fraction from the cytosol, and advantageously also from the nucleus, of the aforementioned yeast. The inactivation step then generates an inactivated yeast or derivative thereof provided with a free amino acids fraction extracted from the cytosol, and advantageously also from the nucleus, of the aforementioned yeast.

Advantageously, this compression does not cause an increase in the temperature of the yeast inside the pressurization chamber, except by a few degrees at most (and in any case without ever reaching temperatures above 30°C) and therefore no deterioration of the yeast proteins occurs due to the heat. Furthermore, the use of a cream or compressed yeast, which has never been dried, further advantageously allows obtaining an inactivated yeast or derivative thereof which has preserved the integrity of the proteins contained within the cell wall.

More specifically, the free amino acids fraction is released from the cytosol, and advantageously also from the nucleus, of the active yeast in the aforementioned inactivation step. In fact, this part of the yeast cell is extremely rich in proteins, amino acids, vitamins and/or other nutritional components and the process in question advantageously allows these components to be made available without the risk of modifying or destroying their molecular structure.

Therefore, following this inactivation step, the inactive yeast or derivative thereof advantageously contains a free amino acids fraction greater than 10 g/kg, preferably greater than 50 g/kg, more preferably equal to or greater than 100 g/kg, even more preferably between 200 g/kg and 500 g/kg.

The process according to the invention further comprises a depressurization step of the pressurization chamber and a step of extracting the inactivated yeast or derivative thereof from the pressurization chamber.

According to a first possible embodiment, in the inactivation step of the process, the pressurized fluid is water, preferably pure water, and the lysis pressure is comprised in a range between 1000 and 7000 bar. In particular, in this case the active yeast is inserted, in the compressed or cream form, into the pressurization chamber of equipment for discontinuous cold pasteurization.

Advantageously, if the pressure is approximately equal to 7000 bar, it is also possible to obtain an autolysed yeast, which is to be considered a derivative of inactivated yeast.

Advantageously, in this first embodiment, before the inactivation step, a step is provided for the insertion of the active yeast into at least one at least partially flexible package and advantageously a step of sealing the package, for example by means of a heat seal, in particular in order to prevent the pressurized water inserted inside the pressurization chamber from entering the package and mixing with the active yeast contained therein. Operationally, the material with which the package is made must be able to withstand the compression imparted by the discontinuous pasteurization equipment without compromising the integrity of the package and/or the seal. Consequently, the package can advantageously be made of food-grade plastic material, such as PET (polyethylene terephthalate), HDPE (high-density polyethylene), LDPE (low-density polyethylene) and PP (polypropylene); or it can be made of flexible aluminum, or even Tetrapak. Furthermore, the package can be made in the form of a bag, a can or a box, or it can be made in the form of a rigid or semi-rigid tray sealed with a flexible covering material, and it can comprise a single layer or multiple layers of flexible material or multiple substrates sealed together.

Furthermore, advantageously, before the sealing step, the package can be subjected to a vacuum-packing step. Thereby, all the active yeast contained in the package is advantageously subjected to the same lysis pressure since, inside the package, there is no gas that can be compressed differently with respect to the active yeast itself. Advantageously, therefore, in the inactivation step the sealed package of active yeast is inserted into the pressurization chamber.

According to a second possible embodiment, in the inactivation step the pressurized fluid is carbon dioxide and the lysis pressure is comprised in a range between 1000 and 1500 bar. In particular, in this case the active yeast in compressed or cream form is inserted into the pressurization chamber of equipment for continuous cold pasteurization.

Advantageously, in this second embodiment, the process does not require packaging the active yeast before inserting it into the pressurization chamber, since the carbon dioxide is not miscible with the yeast and therefore does not dilute the concentration thereof. In other words, in this second embodiment the active yeast is advantageously inserted directly into the pressurization chamber of the continuous cold pasteurization equipment, without a package.

Advantageously, thereby, the carbon dioxide inserted into the pressurization chamber of the equipment **for** continuous cold pasteurization comes into direct contact with the yeast and produces at least a partial breakage of the cell membrane. In fact, carbon dioxide has a corrosive effect on the cell membrane of the yeast and produces an at least partial lysis of the yeast, and therefore a lower lysis pressure is sufficient to obtain an inactivated yeast with characteristics comparable to the inactivated yeast obtained using cold discontinuous pasteurization equipment.

Regardless of the particular embodiment used, in the inactivation step the active yeast is preferably maintained at the lysis pressure for a time comprised between 30 and 600 seconds.

Advantageously, the inactivation step can be performed using the first or second embodiment depending on the type of active yeast made available and on the desired degree of lysis. In fact, discontinuous cold pasteurization allows for a more complete lysis of the active yeast with respect to continuous cold pasteurization.

More clearly, a partial lysis of the yeast molecules is obtained by means of a reduced lysis pressure and in this case it is possible to adopt continuous cold pasteurization equipment, or discontinuous cold pasteurization equipment with low lysis pressures, for example around 3000 bar.

Alternatively, for a breakage of the yeast cells that is as complete as possible, it is possible to provide for adopting continuous cold pasteurization equipment with lysis pressures of approximately 6000 bar. Furthermore, it is advantageously possible to provide for multiple pressurization phases of the active yeast inside the pressurization chamber, in which the pressurization phases can all occur at the same lysis pressure or at increasing lysis pressures, for example a first phase at a lysis pressure of approximately 3000 bar, a second phase at a lysis pressure of approximately 4500 bar and a third phase at a lysis pressure of approximately 6000 bar.

In accordance with an important characteristic of the present invention, the active yeast is yeast in compressed or cream or frozen form, never dried, since experimentally it has been possible to ascertain that only with this type of yeast is the lysis, obtained through discontinuous or continuous cold pasteurization, able to make available the useful components contained inside the yeast cell membrane, i.e. in the cytosol and in the nucleus of the yeast. In particular, it has been found that the availability of unaltered proteins and amino acids present inside the yeast cell membrane, i.e. in the cytosol and in the nucleus of the yeast, significantly influences the efficacy of the inactivated yeast or derivative thereof in the treatment of vine plants to improve the quality of the grapes and the resistance of the plants themselves.

Advantageously, the process according to the invention can provide for further processing steps of the inactivated yeast to obtain one or more derivatives. For example, it is possible to provide for one or more, more or less specific, filtration and/or purification steps of the inactivated yeast to obtain a yeast cell wall, a yeast hull or a yeast extract, which are however enriched with a free amino acids fraction.

As will be seen below, the invention provides a surprising synergic effect due to the use of a selected yeast in the particular form that has never been dried and with a water residue of at least 40%, and to the use of a destructive process of the cell membrane of the same yeast, such as cold pasteurization, to obtain an inactivated yeast or derivative thereof in which the proteins and amino acids contained therein are still available, free and above all unaltered to consequently obtain a surprising effect in the treatment of vine plants, in particular as a soil improver.

As anticipated above, the inactivated yeast or derivative thereof thus obtained is used in the process for inducing the thickening of the skin of grape berries in vine cultivation which is the subject matter of the present invention. According to the invention, the process comprises a dissolution step, in which the inactivated yeast or derivative thereof is dissolved in a solvent, preferably water, to obtain a treatment solution.

Furthermore, the process comprises a distribution step, in which the treatment solution is spread on at least one vine plant, preferably on all the vine plants, of the vine cultivation.

In particular, in the distribution step, the treatment solution is distributed on the surface of the leaves and/or grapes of the vine plant, preferably both.

Furthermore, the distribution step involves the absorption of the free amino acids fraction extracted from the cytosol, and advantageously also from the nucleus, of the yeast by the aforesaid at least one vine plant. In particular, the amino acids are absorbed by the leaves and/or grapes of the vine plant. In more detail, the free amino acids fraction, which is not only more available with respect to that present in known inactivated yeasts (while they are almost totally absent in their cell walls), but also remains unaltered with respect to those present in active yeasts, is absorbed by the vine plants more quickly and in greater quantities.

As will be demonstrated below, the effects of such absorption are surprisingly those of increasing the thickness of the skin of grape berries, significantly improving the technological and phenolic maturity of the grapes, as well as the productivity and resistance of the vine plant to fungal attacks.

According to the preferred embodiment of the invention, in the dissolution step the inactivated yeast or derivative thereof is dissolved in the solvent with a concentration comprised between 0.5 and 2 g/l, preferably between 0.8 and 1.6 g/l, more preferably between 0.8 and 1.4 g/l, even more preferably between 1 and 1.2 g/l. Furthermore, advantageously, in the distribution step a quantity of treatment solution comprised between 500 and 1200 liters is distributed for each cultivation hectare, preferably comprised between 500 and 1100 1/ha, more preferably comprised between 600 and 1100 1/ha, even more preferably comprised between 600 and 1000 1/ha. In any case, the dissolution step and the distribution step are advantageously carried out so as to distribute on the vine plants a quantity of inactivated yeast or derivative thereof comprised between 0.8 and 1.2 kg for each cultivation hectare, preferably comprised between 0.9 and 1.1 kg/ha, more preferably approximately 1 kg/ha. Preferably, the distribution step is performed at least twice, in particular at a time interval of at least 4 days, preferably comprised between 5 and 15 days, more preferably comprised between 6 and 12 days, even more preferably approximately 7 days.

In particular, in accordance with a first embodiment example, particularly suitable for treating vine plants with red berried grape varieties, it was discovered that it is particularly advantageous to perform the distribution step twice at an interval of approximately 7 days.

In particular, in accordance with a second embodiment example, particularly suitable for treating vine plants with white berried grape varieties, it was discovered that it is particularly advantageous to perform the distribution step three times at an interval of approximately 7 days each.

Advantageously, the distribution step is carried out after the phenological phase of budding of the vine plants and more preferably after the phenological phase of fruit setting.

Furthermore, the distribution step is preferably carried out at least in part before the phenological phase of ripening and more preferably at least in part before and/or during the phenological phase of veraison of the vine cultivation plants.

The present invention further relates to the use of an inactivated yeast or derivative thereof for treating a vine cultivation, in particular for inducing thickening of the skin of grape berries. In particular, the inactivated yeast or derivative thereof is used dissolved in a treatment solution to be distributed on at least one vine plant of the vine cultivation.

Furthermore, in accordance with the idea underlying the invention, the inactivated yeast or derivative thereof contains a free amino acids fraction, preferably present in a quantity greater than 10 g/kg, extracted from the cytosol of an active yeast for winemaking use, not dried, with a water residue of at least 40% by mass, by means of and inactivation with a lysis pressure greater than 1000 bar.

More, in detail, the inactivation is carried out by inserting the active yeast and a pressurized fluid into a pressurization chamber. In particular, the pressurized fluid has a lysis pressure greater than 1000 bar, to subject the active yeast to the lysis pressure with consequent breakage of the cell structure of the active yeast and obtain an inactivated yeast or derivative thereof provided with a free amino acids fraction, advantageously released from the cytosol, and advantageously also from the nucleus, of the active yeast during inactivation.

Preferably, the inactive yeast or derivative thereof contains a free amino acids fraction greater than 50 g/kg, more preferably equal to or greater than 100 g/kg, even more preferably between 200 g/kg and 500 g/kg. Advantageously, the inactive yeast or derivative thereof is distributed on the vine plants in a quantity comprised between 0.8 and 1.2 kg for each cultivation hectare, preferably between 0.9 and 1.1 kg/ha, more preferably about 1 kg/ha.

Advantageously, the specific methods for obtaining the inactivated yeast or derivative thereof and using it in the treatment of a vine cultivation can be in whole or in part the same as those already described above and therefore will not be discussed further below.

As anticipated, the treatment of the vine cultivation using inactivated yeast or a derivative thereof is responsible for several positive actions that determine a qualitative improvement of the grapes and the wines obtained therefrom. These positive actions can concern increase of skin thickness, improved phenolic and aromatic ripening, greater pleasantness in the taste of wines obtained from the treated grapes, increased protection of the grapes during the ripening phase, increased resistance of the vine plants to fungal attacks, in particular downy mildew, powdery mildew and botrytis.

These positive effects, due to the presence of amino acids released in the inactivation from the cytosol, and advantageously also from the nucleus, of the yeast, unaltered thanks to the low temperature process, and then absorbed by the vine plants, can be deduced from the experimental tests described below and from the attached figures.

Specifically, the tests reported below were performed on vine plants of different varieties treated with different methods: a first method (V1) in accordance with the first embodiment example of the process in question, a second method (V2) in accordance with the second embodiment example of the process in question, a third method (L1) in accordance with a process that uses a first specific product for white berried grapes known on the market, a fourth method (L2) in accordance with a process that uses a second specific product for red berried grapes known on the market and a fifth method (TNT) in accordance with a control procedure, in which no plants are treated.

In more detail, in the first method (V1) according to the first embodiment example of the process, the inactivated yeast (as is) was dissolved in water and distributed twice at an interval of 7-12 days from each other in quantities of 0.8 kg/ha at a time, uniformly spraying the vine plants, in particular the leaves and the grape berries. In particular, the distribution step was performed for the first time during the phenological phase of pre-veraison of the vine plants.

Furthermore, in the second method (V2) according to the second embodiment example (V2) of the process, the inactivated yeast (as is) was distributed three times at an interval of 7-12 days from each other in quantities of 0.8 kg/ha at a time, uniformly spraying the vine plants, in particular the leaves and the grape berries. In particular, the distribution step was performed for the first time during the phenological phase of pre-veraison of the vine plants, and specifically the second and third times coincide with those of the first method (V1). Furthermore, in the third method (L1), a product known in the sector and consisting of fractions of yeasts inactivated by heat treatment was distributed on the white berried vine plants twice at an interval of 7-12 days from each other in quantities of 3 kg/ha at a time, uniformly spraying the vine plants, in particular the leaves and the grape berries. In particular, the distribution step was performed for the first time during the phenological phase of veraison of the vine plants.

Furthermore, in the fourth method (L2), a product known in the sector and consisting of fractions of yeasts inactivated by heat treatment was distributed on the red berried vine plants twice at an interval of 7-12 days from each other in quantities of 1 kg/ha at a time, uniformly spraying the vine plants, in particular the leaves and the grape berries. In particular, the distribution step was performed for the first time during the phenological phase of veraison of the vine plants.

In particular, to compare the five described methods, experiments were carried out with different experimental combinations, each with a particular variety of vine plants (in the figures indicated above outside the parenthesis), a particular growing area (in the figures the winery is indicated with the code C1-C12 and the Italian or foreign region at the top inside the parenthesis) and a particular vintage (in the figures indicated above inside the parenthesis). For each experimental combination, the plants of the cultivation were schematically divided into randomized blocks and each block was treated with one of the aforesaid treatment methods.

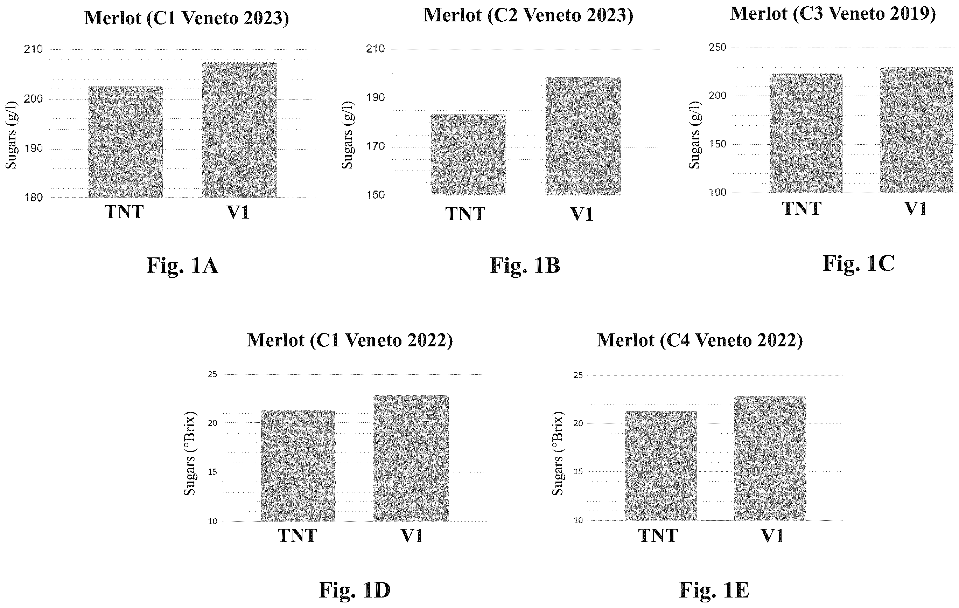

With reference to the attached figures, from the figures in Figures 1A-1D, 2A-2B, 3A-3D, 4, 5A-5C, 6A-6B and 7A-7B it is possible to appreciate the effects of the treatment which is the subject matter of the invention on the technological maturity of the treated grapes.

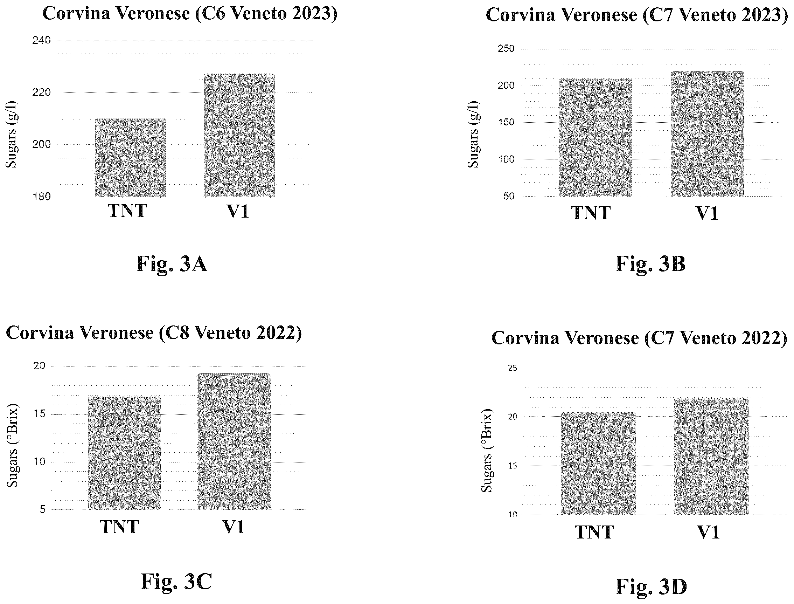

In particular, Figures 1A-1D and 3A-3D show a comparison of the sugar content in the berries of Merlot and Corvina Veronese grape varieties, respectively, collected at the time of harvest from vine plants treated with the first method (V1) and with the fifth method (TNT). From these figures it can be seen that the distribution of inactivated yeast on the vine plants, in particular twice, allows for a greater development of the quantity of sugar in the grapes, measured with known methods in g/l, or in °Brix.

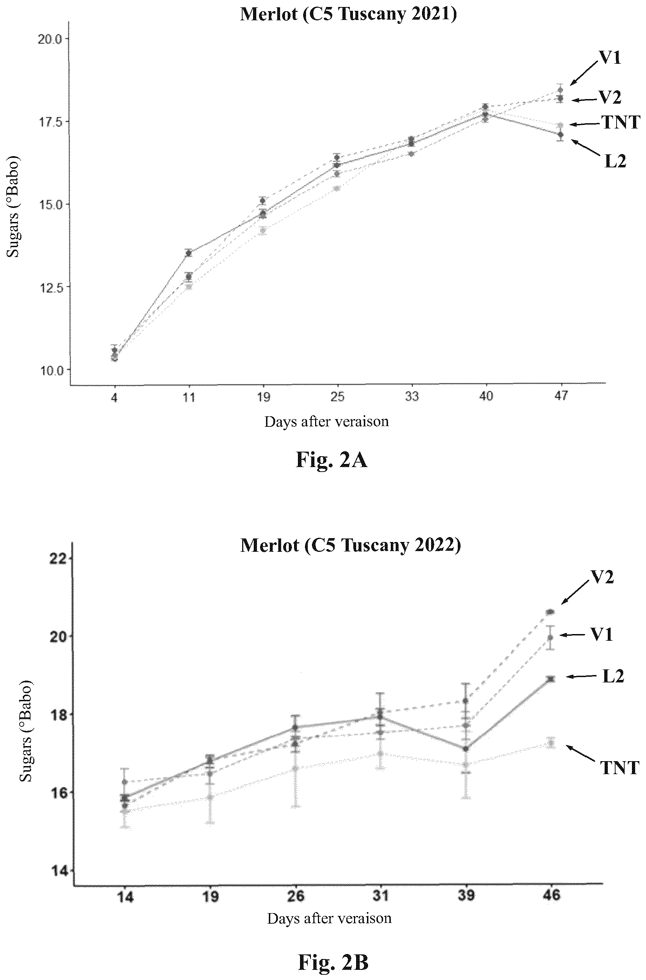

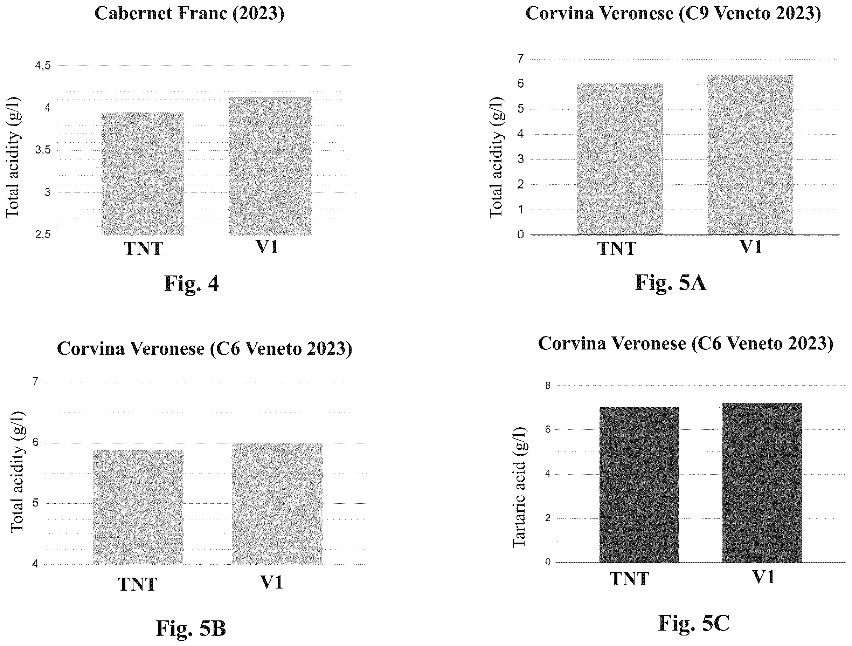

This aspect can also be seen in the figures in Figures 2A and 2B, which show the evolution of the quantity of sugars over time for four treatment methods in Merlot grapes from two different vintages. In both cases, a significant increase in the quantity of sugars is highlighted in the grapes treated with the methods V1 and V2 in accordance with the examples of the process of the invention, especially in the final period of ripening. Furthermore, Figures 4 and 5A-5C show a comparison of the total acid and tartaric acid content in grape musts, respectively in Cabernet Franc and Corvina Veronese grape varieties, collected at the time of harvest from vine plants treated with the first method (V1) and with the fifth method (TNT). From these figures it can be seen that the distribution of inactivated yeast on the vine plants, in particular twice, allows for a greater development of the acidic component, measured with known methods in g/l.

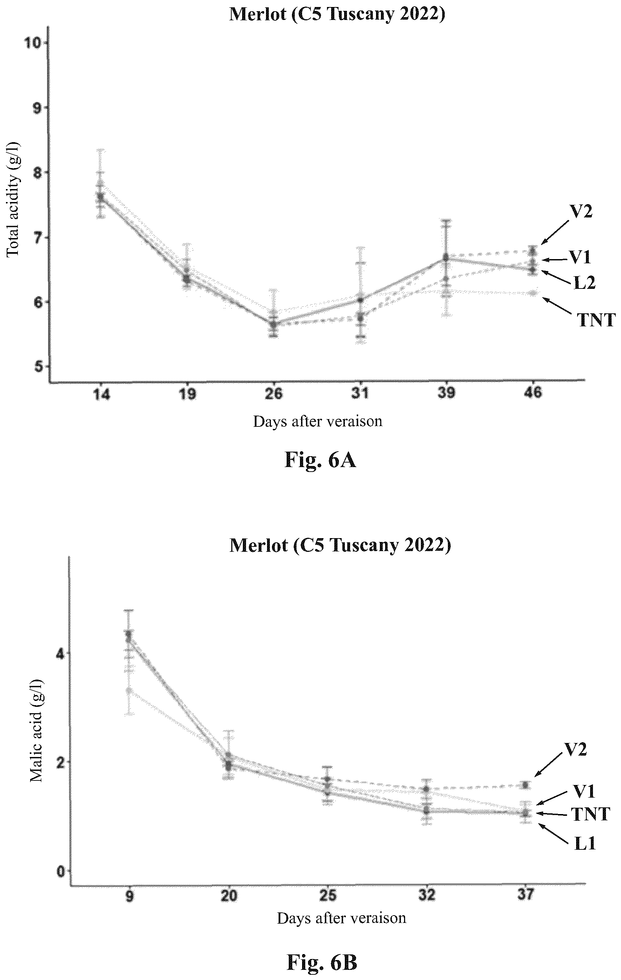

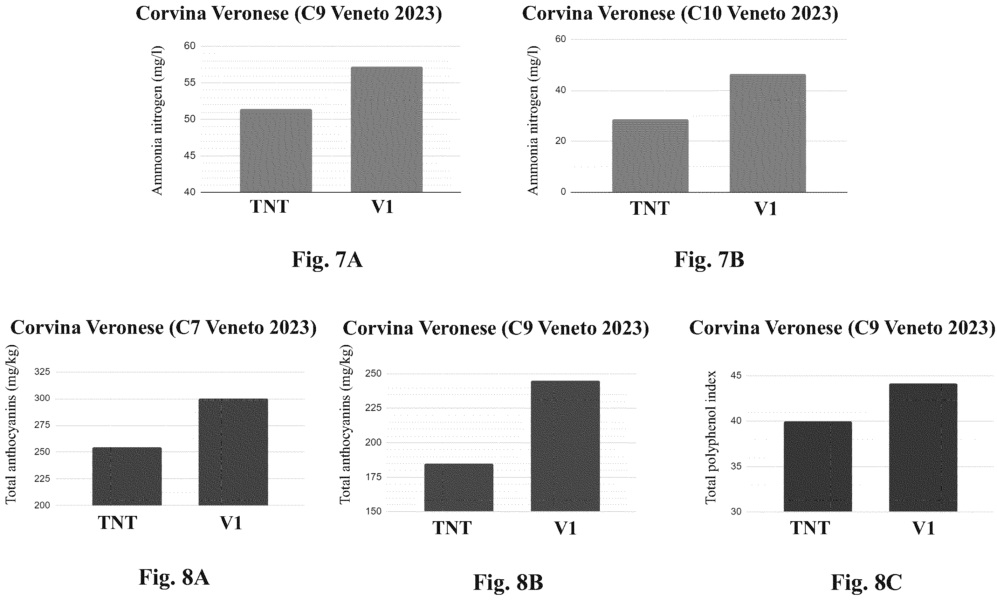

This aspect can also be seen in the figures in Figures 6A and 6B, which respectively show the evolution of total acidity and the quantity of malic acid over time for four treatment methods in Merlot grapes of the same vintage and growing area. In both cases, an increase in total acidity and in the quantity of malic acid is highlighted in the grapes treated especially with the method V2 in accordance with the second example of the process of the invention, especially in the final period of ripening. In this case, therefore, the repetition of the distribution step of the process has a greater effect on the acidity of the musts obtained from the grapes of treated plants. Furthermore, the figures in Figures 7A and 7B show a comparison of the ammonium nitrogen content, measured with known methods in mg/l, contained in Corvina Veronese grapes from two different cultivations, collected at the time of the harvest from vine plants treated with the first method (V1) and with the fifth method (TNT). From these figures it is clear that the distribution of inactivated yeast on the vine plants, in particular twice, allows for a greater development of ammonium nitrogen, which is an essential component for the nutrition of yeasts during the grape fermentation phase.

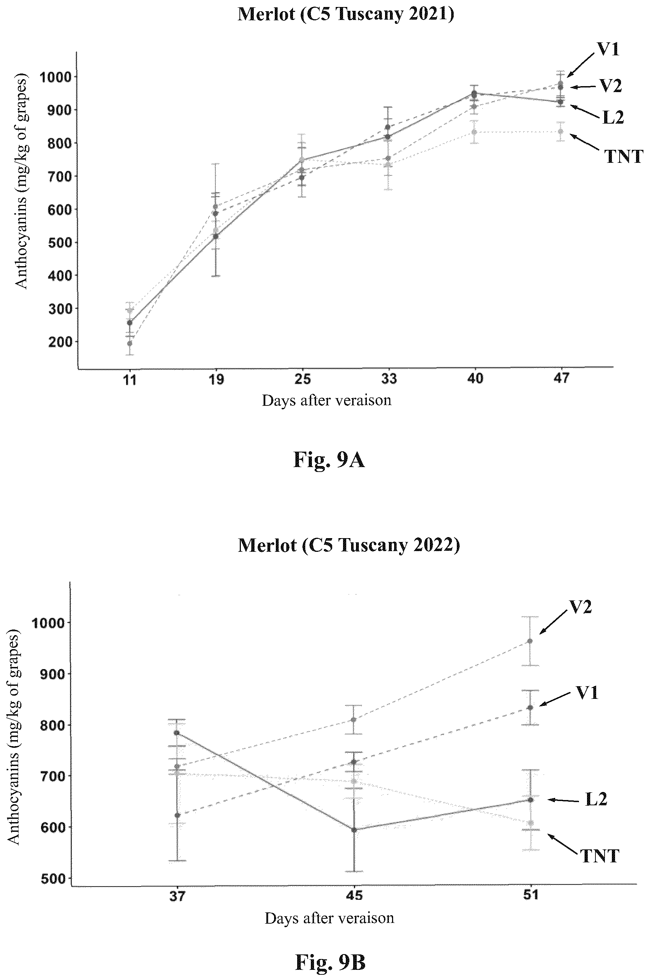

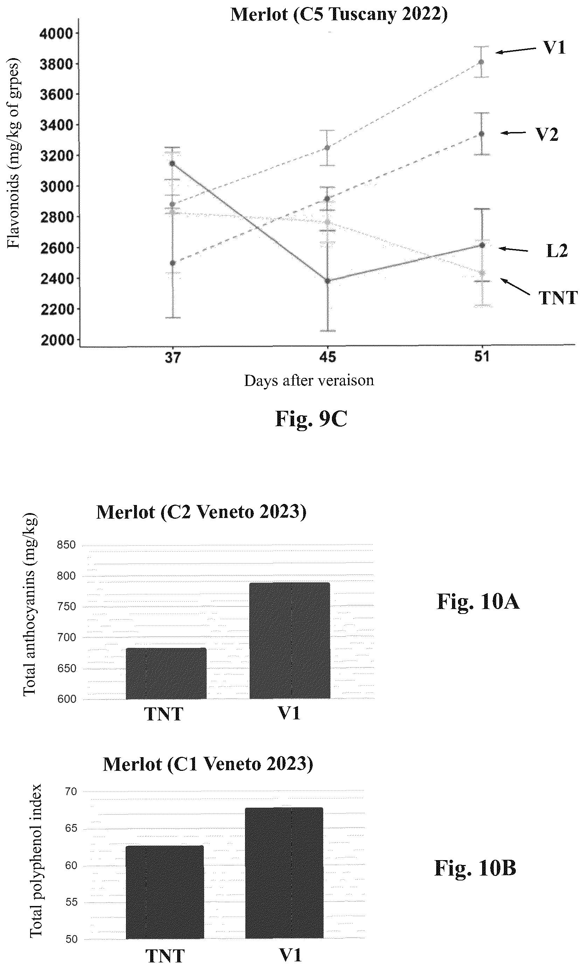

As regards the effects on phenolic maturity, the positive results of the process of the invention are evident from the figures reported in Figures 8A-8C, 9A-9C, 10A-10B, 11A-11F.

In particular, as visible in Figures 8A-8C, 9A-9C and 10A-10B, an increase in the content of total anthocyanins, total polyphenols and flavonoids was found in Merlot and Corvina Veronese grapes from different vintages and cultivations, compared with both untreated grapes (fifth method TNT) and grapes treated with alternative products (fourth method L2). In particular, these quantities were measured in milligrams per kilo of grapes using high-performance liquid chromatography (*Agilent HPLC*)*.*

Furthermore, further tests were performed, the results of which are reported in the following Tables 1 and 2, to detect anthocyanins, flavonoids and polyphenols on wines obtained from Sangiovese and Merlot grapes treated with the first method (V1), with the fourth method (L2) and with the fifth method (TNT). In particular, it is possible to observe that the greater presence of these compounds in the grapes is also reflected in the wines obtained therefrom, thus also improving the color and sensory characteristics, as will be better illustrated below.

**Table 1 - Anthocyanin, flavonoid and polyphenol content of a Sangiovese wine obtained from grapes of treated cultivations (C5 Tuscany 2022)**

| **Compound** | **Unit of measurement** | **TNT** | **V1** |
|---|---|---|---|
| Total anthocyanins | mg/l | 132.80 | 164.61 |
| Total flavonoids | mg/l | 1414.19 | 1566.01 |
| Total polyphenols | mg/l | 1666.97 | 17772.86 |

**Table 2 - Anthocyanin, flavonoid and polyphenol content of a Merlot wine obtained from grapes of treated cultivations (C5 Tuscany 2022)**

| **Compound** | **Unit of measurement** | **TNT** | **L2** | **V1** |
|---|---|---|---|---|
| Total anthocyanins | mg/l | 206 | 308 | 317 |
| Total flavonoids | mg/l | 2263 | 2757 | 3700 |
| Total polyphenols | mg/l | 2264 | 2511 | 2899 |

Comparative tests were also performed using spectrophotometric analysis on Sangiovese and Merlot wines obtained from treated grapes to detect the quantities of anthocyanins, flavonols and hydroxycinnamic acids. The results, reported in Tables 3 and 4, confirm a greater quantity of the aforesaid compounds in the grapes treated with the first method (V1), as they have a corresponding higher absorbance value.

**Table 3 - Spectrophotometric analysis of a Sangiovese wine obtained from grapes of treated cultivations (C5 Tuscany 2021)**

| **Compound detected** | **Wavelength (nm)** | **Absorbance** | | |
|---|---|---|---|---|
| | | **TNT** | **L2** | **V1** |
| Total polyphenols | 280 nm | 40.44 | 41.8 | 42.53 |
| Hydroxycinnamic acids | 320 nm | 14.155 | 14.52 | 14.96 |
| Total flavonols | 365 nm | 4.74 | 4.48 | 4.905 |

**Table 4 - Spectrophotometric analysis of a Merlot wine obtained from grapes of treated cultivations (C5 Tuscany 2021)**

| **Compound detected** | **Wavelength (nm)** | **Absorbance** | | |
|---|---|---|---|---|
| | | **TNT** | **L2** | **V1** |
| Total polyphenols | 280 nm | 31.025 | 32.395 | 33.65 |
| 0Hydroxycinnamic acids | 320 nm | 11.64 | 11.93 | 12.775 |
| Total flavonols | 365 nm | 3.24 | 3.275 | 3.65 |

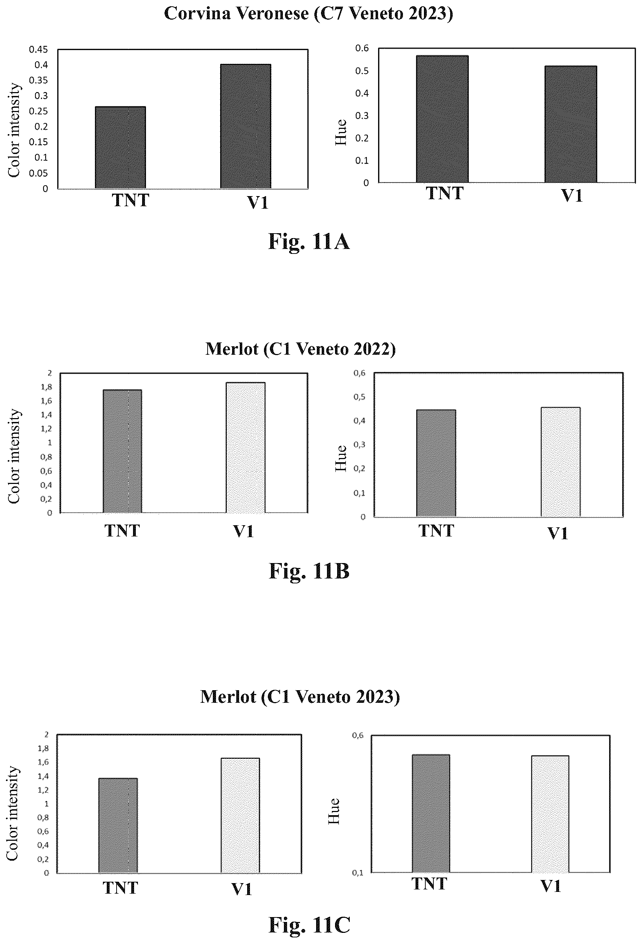

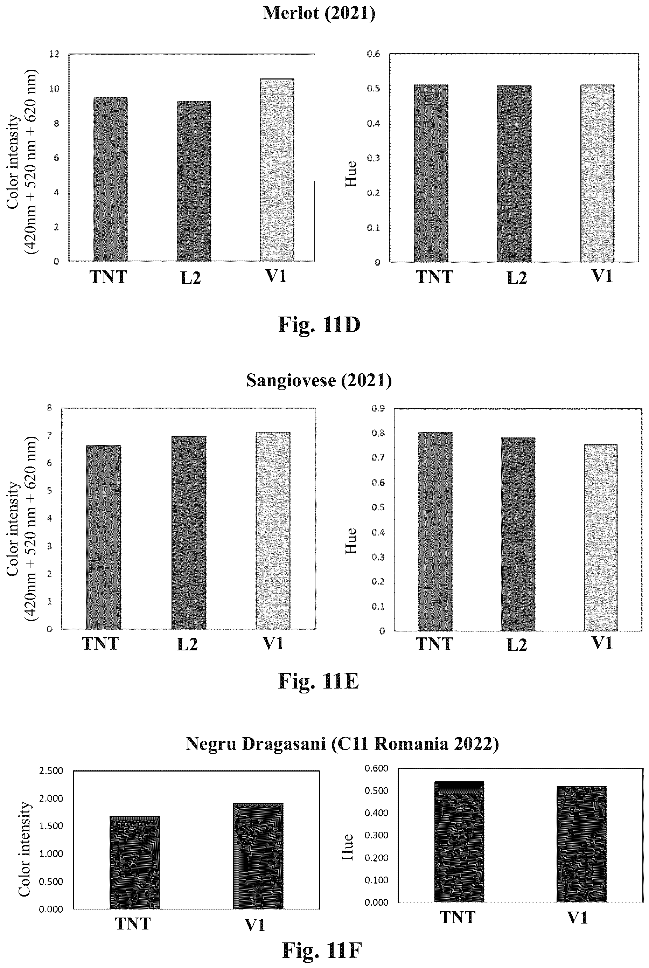

This aspect has an important effect on the color intensity and hue of wines, as visible in the figures shown in Figures 11A-11F. These figures illustrate the results of tests on the color intensity and hue of wines obtained from the vinification of grapes of different varieties, vintages and cultivations treated according to the first method (V1), the fourth method (L2) and the fifth method (TNT). In particular, the color intensity is obtained by adding the absorbance of the wine read at 420 nm, at 520 nm and at 620 nm on a clear wine (centrifuged) under an optical path of 1 cm. Furthermore, the hue is calculated as the ratio between the absorbance of the wine read at 420 nm and at 520 nm.

As can be seen from the figures, the wines obtained from grapes treated with the process of the invention have a greater intensity and a lower hue, improving the color characteristics of the wine, in particular by increasing the red component (absorption at 520 nm due to anthocyanins) compared to the yellow component (absorption at 420 nm due to tannins) which would lead the wine to have an undesired more orange color.

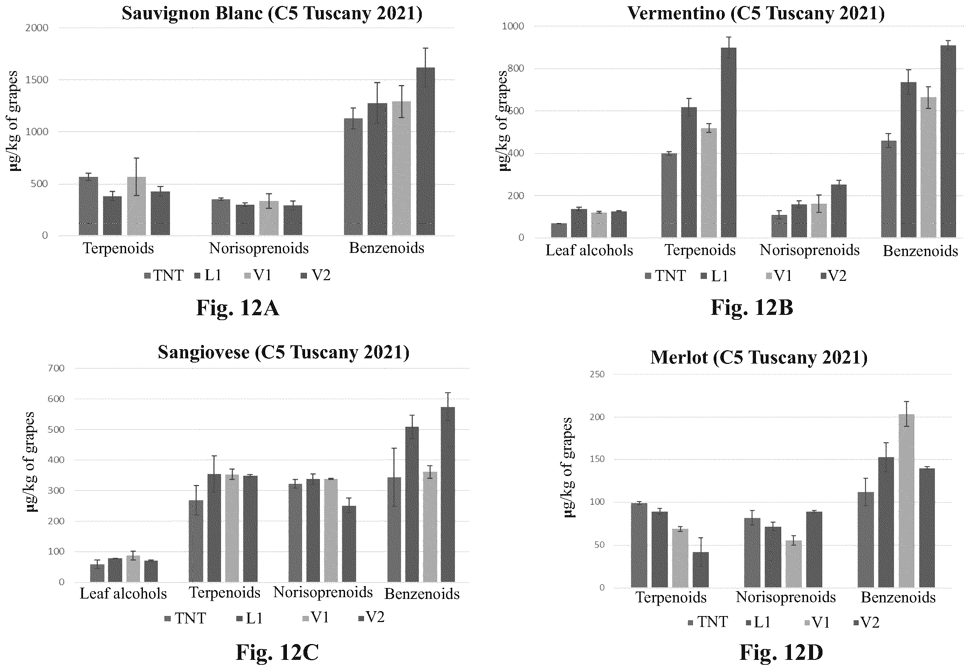

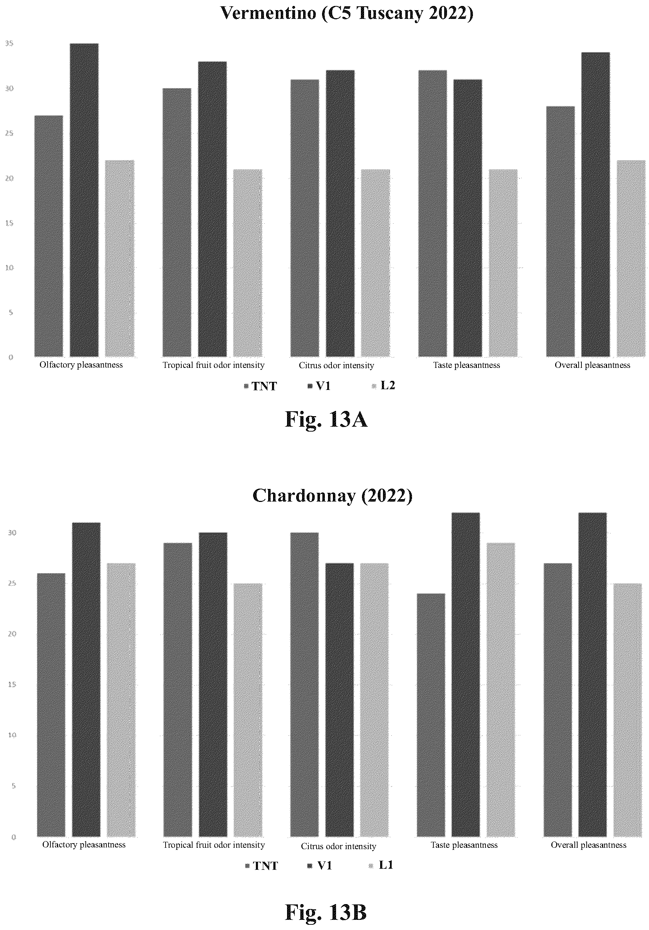

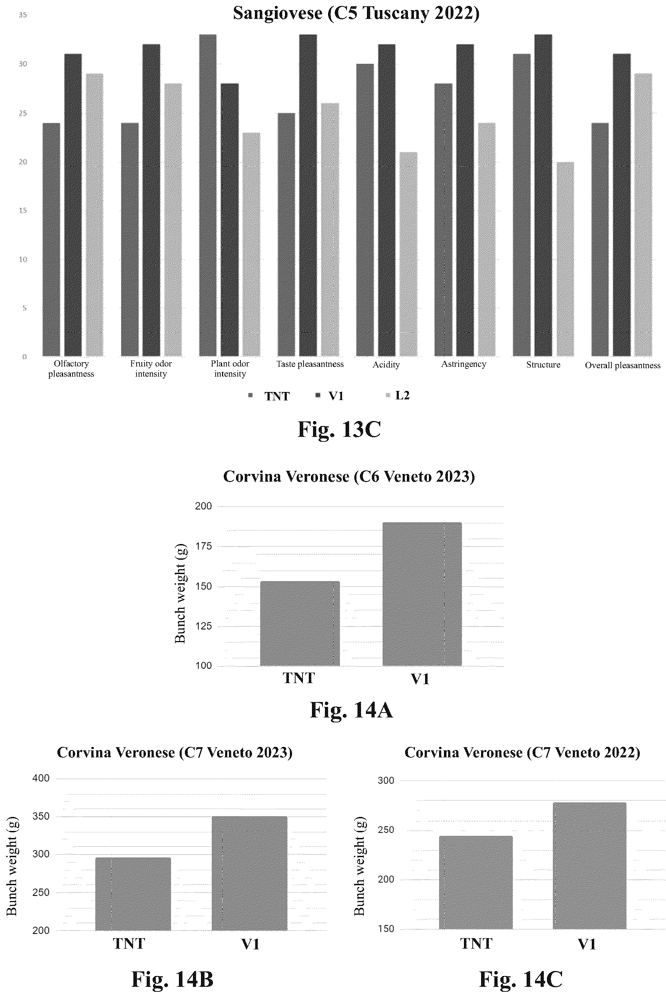

As regards the effect on aromatic maturity, the positive results of the process of the invention are evident from the figures reported in Figures 12A-12D and 13A-13C.

In particular, the figures shown in Figures 12A-12D show the content of different aromatic precursors in the grapes treated with the different methods. It can be noted in general that the processes according to the invention allow obtaining a greater quantity of benzenoids in the treated grapes.

Specifically, the highest production of benzenoids was observed in white berried grapes using the second method (V2), i.e., with the distribution step performed three times. In Vermentino (Figure 12B) in particular, a notable increase can be observed in the quantity of terpenoids and norisoprenoids.

In red berried grapes, the greater effectiveness can vary depending on whether the first or second method (V1, V2) is used, but for both Sangiovese and Merlot there is an improvement due to the processes according to the invention.

Overall, the improvement of the organoleptic properties of the grapes thanks to the process according to the invention are reflected in the sensory characteristics that can be seen from the figures shown in Figures 13A-13C.

These figures show the comparative results of statistical sensory analyses of wines obtained from treated Vermentino, Chardonnay and Sangiovese grapes. From these figures it is possible to note the improvement in almost all the sensorial characteristics of the wines examined.

In accordance with further tests, not reported in the figures, the quantity of additional aromatic precursors was measured in the grapes treated with the first method (V1) and the fifth method (TNT); overall, the tendency was found in the former to have a higher content of thiol and norisoprenoid precursors. In particular, in a Sauvignon Blanc (C12 Friuli Venezia Giulia 2023) values of norisoprenoid precursors of 90 µg/l were measured for V1 grapes, compared to 61 µg/l for TNT grapes, and values of thiol precursors of 82.17 µg/l for V1 grapes, compared to 70.43 µg/l for grapes TNT. The most present thiol precursor in this case was Cy3MH, with a quantity of 1.88 µg/l for V1 grapes, compared to 1.27 µg/l for TNT grapes.

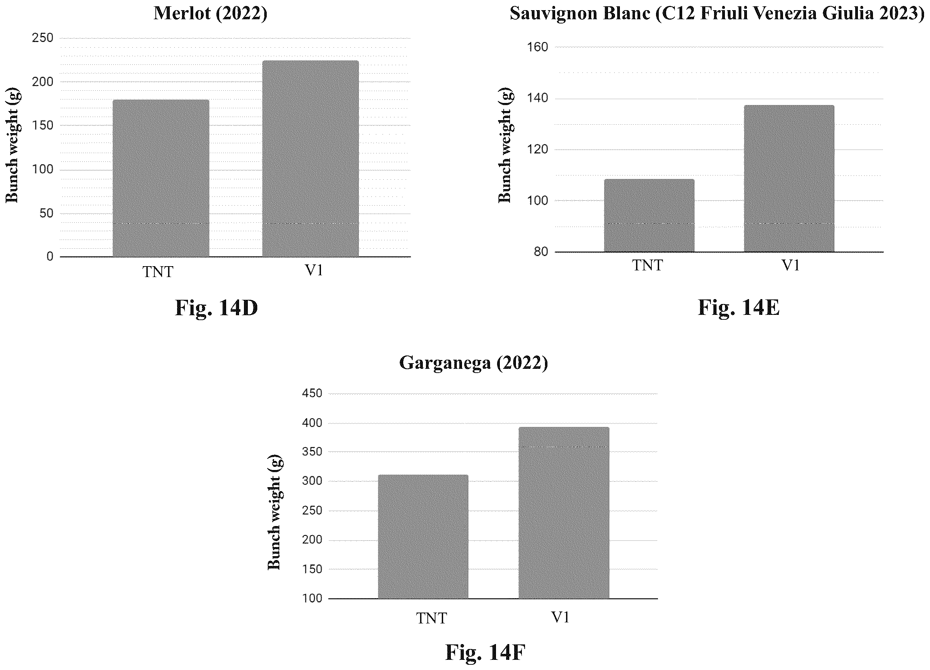

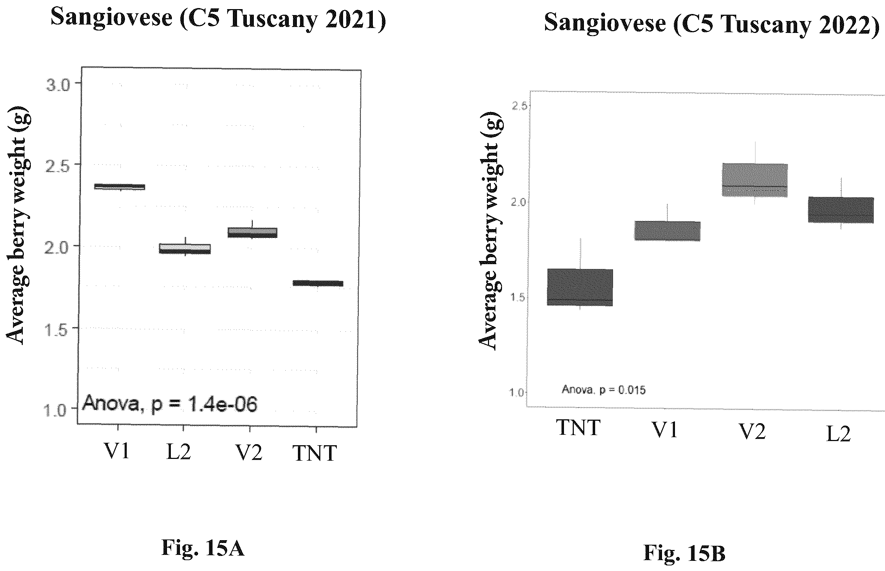

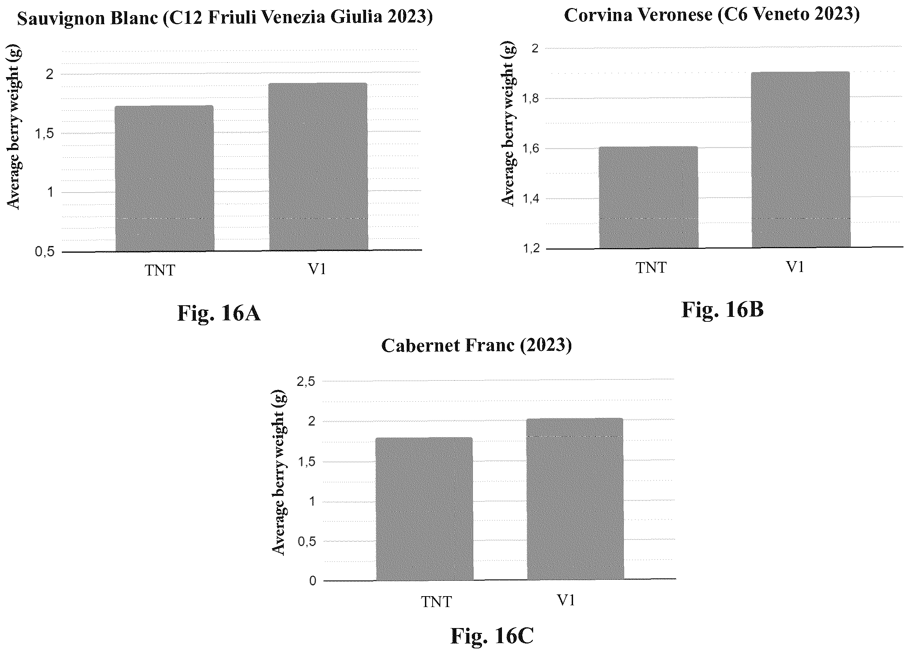

As regards the effect on production parameters, the positive results of the process of the invention are evident from the figures shown in Figures 14A-14F, 15A-15B and 16A-16C. In particular, from these figures it is possible to note that both the average weight of the bunch (Figures 14A-14F) and the average weight of the berry (Figures 15A-15B and 16A-16C) are greater for the grapes treated with the processes according to the invention. Thickness measurements, reported in the following Table 5, were also performed on the thickness of the skin of the harvested berries. In particular, the thickness measurements were initially carried out by placing the grape berries in a freezer at -20 °C, then taking skins from the frozen grapes with a microtome *(Reichert II LG mod. 1130*/*Biocut),* placing them in a 95% Vol alcohol solution for washing and subsequently embedding them in paraffin. The thin portions were placed under observation through a bi-ocular *(Nikon SMZ 25)* with integrated calculation software.

**Table 5 - Skin thickness analysis of Sangiovese, Merlot and Vermentino grapes from treated cultivations (C5 Tuscany 2021)**

| **Variety** | **Treatment** | | |
|---|---|---|---|
| | **TNT** | **V1** | **V2** |
| Sangiovese | 218 µm | 220 µm | 218 µm |
| Merlot | 215 µm | 217 µm | 217 µm |
| Vermentino | 212 µm | 215 µm | 217 µm |

From these analyses, it is possible to observe that with the process and/or use according to the invention it is possible to obtain an average thicker skin, which contributes to maintaining greater integrity of the grapes both during their ripening and during the harvest.

Furthermore, as regards the evaluation of the effect on the resistance of vine plants to fungal attacks, continuous surveys on the spread of downy mildew were carried out throughout the entire season in which the experimental vine plants were grown according to the methods described. In particular, in the vine plants treated with the methods V1 and V2, a diffusion of this fungus was detected that never exceeded 2.0% in the leaves and 25% in the bunches with an attack intensity never exceeding 0.3% in the leaves and 0.8% in the bunches, compared to a diffusion of 5.5 - 35.5% in the leaves and 6.5 - 26.0% in the bunches with an attack intensity of 2.0 - 21.0% in the leaves and 2.6 - 16.6% in the bunches. Therefore, the measured treatment efficiency is higher than 97.9% for leaf treatment and higher than 93.7% for bunch treatment.

This effect has been attributed to the stimulation of the production of organic compounds with antifungal action synthesized by the plant following the process according to the invention. In particular, a higher production of piceid and piceatannol was measured from the plants treated according to the process according to the invention and in particular with the methods V1 and V2.

Therefore, a greater resistance to downy mildew is observed, but thanks to the synthesis of these substances, there are also important improvements in resistance to other fungal attacks such as powdery mildew and botrytis. Further tests were also performed, in which the quantity of amino acids present in a yeast inactivated with the inactivation step according to the present process, and used in the previous embodiment examples of the first method (V1) and the second method (V2), was measured, compared with the quantity of amino acids present in a yeast inactivated with a traditional method present on the market. The results of these tests are reported in Table 6.

**Table 6 - Quantities of amino acids present in inactive yeast**

| | **Unit of measurement** | **Traditional inactivated yeast** | **Inactivated yeast according to the invention** |
|---|---|---|---|
| Dry residue | % | 91 | 1 |
| Total amino acids (AA) | g/kg | 5.3 | 270 |
| Total peptides | g/kg | 35 | 520 |
| NH₄+ | g/kg | 0.03 | 4.60 |
| Soluble not AA o NH₄+ | g/kg | 49.6 | 195.7 |
| Insoluble (cells) | g/kg | 910 | 10 |

| **Amino acids:** | | | |
|---|---|---|---|
| ala | mg/kg | 1114 | 25466 |
| arg | mg/kg | 302 | 12223 |
| asn | mg/kg | 26 | 8605 |
| asp | mg/kg | 26 | 15776 |
| cit | mg/kg | 100 | 1728 |
| gaba | mg/kg | 56 | 7605 |
| gln | mg/kg | 7 | 12660 |
| glu | mg/kg | 2284 | 18972 |
| gly | mg/kg | 117 | 8291 |
| hys | mg/kg | 63 | 5016 |
| ile | mg/kg | 83 | 12963 |
| leu | mg/kg | 114 | 26813 |
| lys | mg/kg | 109 | 15667 |
| orn | mg/kg | 369 | 4569 |
| phe | mg/kg | 83 | 16494 |
| ser | mg/kg | 4 | 13452 |
| si | mg/kg | 96 | 9925 |
| thr | mg/kg | 28 | 9209 |
| trp+met | mg/kg | 68 | 15079 |
| tyr | mg/kg | 75 | 11863 |
| val | mg/kg | 210 | 17277 |

As it can be seen from the comparison of the quantity of amino acids, the process object of the invention allows to obtain a quantity of free amino acids much higher than that of the known inactivated yeasts, since the inactivation step has the effect of freeing the amino acids from the cytosol, and advantageously also from the nucleus, of the yeast cell without modifying the molecular structure of the amino acids themselves. Furthermore, the process advantageously makes the inactive yeast more soluble in water than the cell walls used in the prior art.

Finally, further tests were carried out, in which the quantity of vitamins present in a yeast inactivated with the inactivation step was measured in accordance with the present process, and used in the previous embodiment examples of the first method (V1) and the second method (V2). The results of these tests are reported in Table 7.

**Table 7 - Quantities of vitamins present in inactivated yeast detected**

| **Compound** | **Unit of measurement** | **Quantity** |
|---|---|---|
| Vitamina B2 (riboflavina) | mg/kg | 70 |
| Vitamina B6 | mg/kg | 55.4 |
| Vitamina D3 | mg/kg | 40.2 |

Therefore, the yeast inactivated according to the process in question is also rich in vitamins which advantageously contribute to improving the resistance and organoleptic properties of the grapes.

From all the results described above, it is clear that the process of the present invention produces a notable improvement in the characteristics of the grapes with respect to the same ones not treated or treated with known products.

Therefore, the invention thus conceived achieves the pre-set objects.

## Claims

1. Process for inducing thickening of the skin of grape berries in a vine cultivation, which comprises a plurality of vine plants; which process comprises:
- a step of arranging an active yeast for winemaking use, not dried, with water residue of at least 40% by mass;
- an inactivation step, in which in a pressurization chamber, said active yeast and a pressurized fluid are inserted; said pressurized fluid having a lysis pressure higher than 1000 bar, in order to subject said active yeast to said lysis pressure with consequent breakage of the cell structure of said active yeast and release of a free amino acids fraction from the cytosol of said yeast; said inactivation step generating an inactivated yeast or derivative thereof provided with a free amino acids fraction extracted from the cytosol of said yeast;
- a step of depressurizing said pressurization chamber;
- a step of extracting said inactivated yeast or derivative thereof from said pressurization chamber;
- a dissolution step, in which said inactivated yeast or derivative thereof is dissolved in a solvent in order to obtain a treatment solution;
- a distribution step, in which said treatment solution is spread on at least one vine plant of said vine cultivation with adsorption of said free amino acids fraction extracted from the cytosol of said yeast by at least one vine plant.

2. Process according to claim 1, **characterized in that** said inactive yeast or derivative thereof contains a free amino acids fraction greater than 10 g/kg, preferably greater than 50 g/kg, more preferably equal to or greater than 100 g/kg, even more preferably between 200 g/kg and 500 g/kg.

3. Process according to claim 1 or 2, **characterized in that** said dissolution step and said distribution step are carried out so as to distribute on the vine plants a quantity of inactivated yeast or derivative thereof comprised between 0.8 and 1.2 kg for each cultivation hectare, preferably comprised between 0.9 and 1.1 kg/ha, more preferably approximately 1 kg/ha.

4. Process according to any one of the preceding claims, **characterized in that** in said dissolution step, said inactivated yeast or derivative thereof is dissolved in said solvent with concentration comprised between 0.5 and 2 g/l, preferably between 0.8 and 1.6 g/l, more preferably between 0.8 and 1.4 g/l, still more preferably between 1 and 1.2 g/l.

5. Process according to any one of the preceding claims, **characterized in that** in said distribution step, a quantity of treatment solution is distributed that is comprised between 500 and 1200 liters for each cultivation hectare, preferably comprised between 500 and 1100 l/ha, more preferably comprised between 600 and 1100 1/ha, still more preferably comprised between 600 and 1000 l/ha.

6. Process according to any one of the preceding claims, **characterized in that** said distribution step is executed at least twice, preferably at a time interval of at least 4 days, more preferably comprised between 5 and 9 days, still more preferably about 7 days.

7. Process according to any one of the preceding claims, **characterized in that** said distribution step is executed after the phenological step of budding of the plants of said vine cultivation and at least partly before the phenological step of maturation of the plants of the vine cultivation.

8. Process according to any one of the preceding claims, **characterized in that** before said inactivation step, the following is provided for:
- a step of inserting said active yeast in at least one package that is at least partly flexible;
- a step of sealing said package.

9. Process according to any one of the preceding claims, **characterized in that** in said inactivation step said pressurized fluid is water and said lysis pressure is comprised in an interval between 1000 and 6500 bar.

10. Process according to any one of the preceding claims, **characterized in that**, in said inactivation step, said pressurized fluid is carbon dioxide and said lysis pressure is comprised in an interval between 1000 and 1500 bar.

11. Process according to any one of the preceding claims, **characterized in that**, in said inactivation step, said active yeast is maintained at said lysis pressure for a time comprised between 30 and 600 seconds.

12. Process according to any one of the preceding claims, **characterized in that** said inactivated yeast or derivative thereof is selected from among inactivated yest as such and autolysed yeast.

13. Use of an inactivated yeast or derivative thereof for treating a vine cultivation, in particular for inducing thickening of the skin of grape berries, in which said inactivated yeast or derivative thereof is used, dissolved in a treatment solution to be distributed on at least one vine plant of said vine cultivation;
said inactivated yeast or derivative thereof containing a free amino acids fraction, present in a quantity greater than 10 g/kg and extracted from the cytosol of an active yeast for oenological use, not dried, with water residue of at least 40% by mass, by inactivation with a lysis pressure higher than 1000 bar.

14. Use of an inactivated yeast or derivative thereof according to claim 13, **characterized in that** said inactivated yeast or derivative thereof is distributed on the vine plants in a quantity comprised between 0.8 and 1.2 kg for each cultivation hectare, preferably comprised between 0.9 and 1.1 kg/ha, more preferably approximately 1 kg/ha.

15. Use of an inactivated yeast or derivative thereof according to claim 13 or 14, **characterized in that** said inactive yeast or derivative thereof contains a free amino acids fraction greater than 50 g/kg, more preferably equal to or greater than 100 g/kg, even more preferably between 200 g/kg and 500 g/kg.
